# EUROPEAN PATENT APPLICATION

(11) **EP 4 604 130 A1**
(43) Date of publication of application: **20.08.2025**
(21) Application number: 25157344.0
(22) Date of filing: 12.02.2025
(51) Int. Cl.: G16C 20/30, G16C 20/70, G16C 60/00

(54) **METHOD FOR PREDICTING CHARACTERISTIC OF POLYMER COMPOSITE MATERIALS BASED ON MATERIAL AND DEVICE THEREOF**

(30) Priority: 15.02.2024 KR 20240022079
(71) Applicant: SK Geo Centric Co., Ltd., Seoul 03188 (KR); SK innovation Co., Ltd., Seoul 03188 (KR)
(72) Inventor: JEON, Chan Woong, 03161 Seoul (KR); KWON, June Haan, 03161 Seoul (KR); KIM, Duk Hee, 03161 Seoul (KR); KIM, Seok Jun, 03161 Seoul (KR); KIM, Sun Ryong, 03161 Seoul (KR); KIM, Jeong Hwan, 34124 Daejeon (KR); KIM, Chan Woo, 03161 Seoul (KR); LEE, Joo Pyung, 03161 Seoul (KR); LEE, Hee Eun, 34124 Daejeon (KR); JUNG, Hyun Jung, 34124 Daejeon (KR)
(74) Representative: Thoma, Michael

(57) **Abstract**

According to various embodiments of the present disclosure, a method for predicting characteristics of a polymer composite material and a device thereof may be provided, wherein the method comprises: inputting a recipe including two or more materials containing at least one polymer and a mixing ratio for each of the two or more materials; predicting properties of the polymer composite material according to the recipe based on a recipe and property prediction model; and outputting the properties of the polymer composite material.

## Description

### [BACKGROUND OF THE INVENTION]

### 1. Field of the Invention

The present disclosure relates to a method for predicting characteristics of polymer composite materials and a device thereof.

### 2. Description of the Related Art

A polymer composite material is used in various industrial fields, and in order to predict properties according to recipes during the development and synthesis thereof, experiments, simulations, and data-based approaches are comprehensively utilized.

The current techniques use methods which are still trial and error methods in order to predict the properties of the produced polymer composite material according to the recipes, for example, a method of collecting property data by trying the prediction while changing various composition ratios and process conditions in a laboratory, then inferring and improving the properties of the material based on the collected property data is used.

Accordingly, attempts are being made to predict the properties of the polymer composite material according to the recipes using a model trained based on the artificial intelligence currently developed, but it takes a lot of costs and time to perform training of the commercially available artificial intelligence model, and in particular, there are limitations due to a reason that it is difficult to consider numerous variables of the materials forming the recipes and interactions therebetween.

### [SUMMARY OF THE INVENTION]

It is an object of the present disclosure to provide a method for predicting characteristics of a polymer composite material and a device thereof.

Problems to be solved through various embodiments are not limited to the above-described problem, and other problems not described above will be clearly understood by those skilled in the art from the following description.

To achieve the above object, according to an aspect of the present disclosure, there is provided a method for predicting characteristics of a polymer composite material, which includes: inputting a recipe including two or more materials containing at least one polymer and a mixing ratio for each of the two or more materials; predicting properties of the polymer composite material according to the recipe based on a recipe and property prediction model; and outputting the properties of the polymer composite material.

Here, the training of the property prediction model may be performed through the steps of acquiring a plurality of learning recipes including two or more learning materials containing at least one polymer and a mixing ratio for each of the two or more learning materials, and properties of a plurality of learning polymer composite materials according to each of the plurality of learning recipes, as a dataset; and performing training of the property prediction model to predict properties of the learning polymer composite material according to each of the plurality of learning recipes based on the properties of the learning materials included in each of the plurality of learning recipes, and the properties of the plurality of learning polymer composite materials.

Here, the step of performing training of the property prediction model may include: extracting, from each of the plurality of learning recipes, a first preset learning specific property among properties possessed by each of at least one learning specific material, and a second preset learning specific property among the properties of the learning polymer composite material; and performing training of the property prediction model for each of the plurality of learning recipes based on the plurality of learning recipes including the plurality of first learning specific properties and the second learning specific properties, wherein the specific material includes at least one material of a polymer, talc, and a polyolefin elastomer.

Here, the step of predicting properties of the polymer composite material may include: extracting at least one specific property among a plurality of properties possessed by each of at least one specific material among the two or more materials; and predicting properties of the polymer composite material using the two or more materials and the at least one specific property of the at least one specific material as inputs to the property prediction model.

In addition, according to another aspect of the present disclosure, there is provided a method for predicting characteristics of a polymer composite material, which includes: inputting a recipe including two or more materials containing at least one polymer and a mixing ratio for each of the two or more materials; predicting at least one attribute for each of the two or more materials based on the two or more materials and a property prediction model; predicting properties of the polymer composite material according to the recipe based on the at least one attribute for each of the two or more materials and the property prediction model; and outputting the properties of the polymer composite material.

Here, the training of the property prediction model may be performed through the steps of: acquiring a plurality of learning recipes including two or more learning materials containing at least one polymer and a mixing ratio for each of the two or more learning materials, and properties of a plurality of learning polymer composite materials according to each of the plurality of learning recipes, as a dataset; and performing training of the property prediction model based on properties of the learning materials included in each of the plurality of learning recipes, attributes of the learning materials, and the properties of the plurality of learning polymer composite materials.

Here, the step of performing training of the property prediction model may include: training to infer attributes of the learning materials included in each of the plurality of learning recipes based on the properties of the learning materials included in each of the plurality of learning recipes; and training to predict the properties of the learning polymer composite material according to each of the plurality of learning recipes based on the attributes of the learning materials included in each of the plurality of learning recipes.

Here, the step of performing training of the property prediction model further may include extracting, from each of the plurality of learning recipes, a first preset learning specific property among properties possessed by each of at least one learning specific material, and a second preset learning specific property among the properties of the learning polymer composite material, wherein the step of training to infer attributes of the learning materials performs training to infer attributes of the learning materials included in each of the plurality of learning recipes based on the first learning specific property included in each of the plurality of learning recipes, the step of training to predict properties of the learning polymer composite material performs training to predict the second specific property of the learning polymer composite material according to each of the plurality of learning recipes based on the attributes of the learning materials included in each of the plurality of learning recipes, and the specific material includes at least one material of a polymer, talc, and a polyolefin elastomer.

Here, the step of predicting properties of the polymer composite material may include: extracting at least one specific property among a plurality of properties possessed by each of at least one specific material among the two or more materials; and predicting properties of the polymer composite material using the two or more materials and the at least one specific property of the at least one specific material as inputs to the property prediction model.

Further, according to another aspect of the present disclosure, there is provided a device for predicting characteristics of a polymer composite material, which includes: an information input unit configured to input a recipe including two or more materials containing at least one polymer and a mixing ratio for each of the two or more materials; an information prediction unit configured to predict properties of the polymer composite material according to the recipe based on a recipe and property prediction model; and a result output unit configured to output the properties of the polymer composite material.

Here, the device may further include a model learning unit configured to: acquire a plurality of learning recipes including two or more learning materials containing at least one polymer and a mixing ratio for each of the two or more learning materials, and properties of a plurality of learning polymer composite materials according to each of the plurality of learning recipes, as a dataset; and perform training of the property prediction model to predict the properties of the learning polymer composite material according to each of the plurality of learning recipes based on the properties of the learning materials included in each of the plurality of learning recipes, and the properties of the plurality of learning polymer composite materials, wherein the property prediction model is trained through the model learning unit.

Here, the model learning unit may be configured to: extract, from each of the plurality of learning recipes, a first preset learning specific property among properties possessed by each of at least one learning specific material, and a second preset learning specific property among the properties of the learning polymer composite material; and perform training of the property prediction model for each of the plurality of learning recipes based on the plurality of learning recipes including the plurality of first learning specific properties and the second learning specific properties, wherein the specific material includes at least one material of a polymer, talc, and a polyolefin elastomer.

Here, the information prediction unit may be configured to: extract at least one specific property among a plurality of properties possessed by each of at least one specific material among the two or more materials; and predict the properties of the polymer composite material using the two or more materials and the at least one specific property of the at least one specific material as inputs to the property prediction model.

Furthermore, according to another aspect of the present disclosure, there is provided a device for predicting characteristics of a polymer composite material, which includes: an information input unit configured to input a recipe including two or more materials containing at least one polymer and a mixing ratio for each of the two or more materials; an information prediction unit configured to predict at least one attribute for each of the two or more materials based on the two or more materials and a property prediction model, and predict properties of the polymer composite material according to the recipe based on the at least one attribute for each of the two or more materials and the property prediction model; and a result output unit configured to output the properties of the polymer composite material.

Here, the property prediction model may include: a property-attribute prediction model configured to predict the at least one attribute; and an attribute-property prediction model configured to predict the properties of the polymer composite material.

Here, the device may further include a model learning unit configured to: acquire a plurality of learning recipes including two or more learning materials containing at least one polymer and a mixing ratio for each of the two or more learning materials, and properties of a plurality of learning polymer composite materials according to each of the plurality of learning recipes, as a dataset; and perform training of the property prediction model based on properties of the learning materials included in each of the plurality of learning recipes, attributes of the learning materials, and the properties of the plurality of learning polymer composite materials, wherein the property prediction model is trained through the model learning unit.

Here, the model learning unit may perform training of: the property-attribute prediction model to infer attributes of the learning materials included in each of the plurality of learning recipes based on the properties of the learning materials included in each of the plurality of learning recipes, and the attribute-property prediction model to predict properties of the learning polymer composite material according to each of the plurality of learning recipes based on the attributes of the learning materials included in each of the plurality of learning recipes.

Here, the model learning unit may be configured to: extract, from each of the plurality of learning recipes, a first preset learning specific property among properties possessed by each of at least one learning specific material, and a second preset learning specific property among the properties of the learning polymer composite material; perform training of the property-attribute prediction model to infer attributes of the learning materials included in each of the plurality of learning recipes based on the first learning specific property included in each of the plurality of learning recipes; and perform training of the attribute-property prediction model to predict the second specific property of the learning polymer composite material according to each of the plurality of learning recipes based on the attributes of the learning materials included in each of the plurality of learning recipes, wherein the specific material includes at least one material of a polymer, talc, and a polyolefin elastomer.

Here, the information prediction unit may be configured to: extract at least one specific property among a plurality of properties possessed by each of at least one specific material among the two or more materials; and predict the properties of the polymer composite material using the two or more materials and the at least one specific property of the at least one specific material as inputs to the property prediction model.

According to various embodiments, the method for predicting characteristics of a polymer composite material and the device thereof may provide an environment capable of quickly checking changes in the properties of the polymer composite material prepared during synthesis only by changing the materials and the mixing ratio of the materials.

According to various embodiments, the method for predicting characteristics of a polymer composite material and the device thereof allow users to predict the characteristics of the polymer composite material prepared during synthesis according to the mixing ratio of the materials included in the recipes without performing the material synthesis and property measurement processes for the polymer composite material, thereby significantly reducing the time and costs required for development of materials.

### [BRIEF DESCRIPTION OF THE DRAWINGS]

The above and other objects, features and other advantages of the present disclosure will be more clearly understood from the following detailed description taken in conjunction with the accompanying drawings, in which:
FIG. 1 is a block diagram schematically illustrating the configuration of a device according to an embodiment;
FIG. 2 is a flowchart illustrating the flow of an operation of predicting characteristics of a polymer composite material in the device according to an embodiment;
FIG. 3 is a view schematically illustrating a property prediction model to output properties of the polymer composite material using properties of the materials included in a recipe as an input in the device according to an embodiment;
FIG. 4 is a flowchart illustrating the flow of an operation of training the property prediction model based on a deep learning algorithm in the device according to an embodiment;
FIG. 5 is a flowchart illustrating the flow of an operation of predicting characteristics of the polymer composite material in the device according to an embodiment;
FIG. 6 is a view schematically illustrating an operation of the property prediction model to predict attributes of the materials using the properties of the materials included in the recipe as an input, and output properties of the polymer composite material using the attributes of the materials as an input in the device according to an embodiment;
FIG. 7 is a flowchart illustrating the flow of an operation of training the property prediction model based on the deep learning algorithm in the device according to an embodiment of the present disclosure; and
FIG. 8 is a flowchart illustrating the flow of the detailed operation of training the property prediction model based on the deep learning algorithm in the device according to an embodiment of the present disclosure.

### [DETAILED DESCRIPTION OF THE INVENTION]

Hereinafter, embodiments will be described in detail with reference to the accompanying drawings. However, since various changes may be made in the embodiments, the scope of the patent disclosure is not limited or restricted by these embodiments. It should be understood that all modifications, equivalents, and alternatives for the embodiments are included in the scope of the present disclosure.

It will be understood that when a component is described to as being "connected," "combined" or "coupled" to another component, the component may be directly connected or coupled the another component, but it may be "connected," "combined" or "coupled" to the another component intervening another component may be present.

Further, in describing the components of the embodiment, the meaning of "or" may mean each of the components, may mean two or more of the components, or may mean all of the components. For example, it should be understood that the expressions "a, b or c" represent any one of "a," "b," "c," "a and b," "a and c," "b and c," and "a, b and c."

Components included in one embodiment and components including common functions will be described using the same names in other embodiments. The description given in one embodiment may be applied to other embodiments, and therefore will not be described in detail within the overlapping range, unless there is a description opposite thereto.

The device and/or 'data' processed by the device may be expressed in terms of 'information'. Here, the information may be used as a concept including the data.

The present disclosure relates to a method for predicting characteristics of a polymer composite material and a device thereof. To describe in more detail, a method for predicting properties of a polymer composite material prepared (or synthesized) based on materials of input recipes and a device thereof will be described.

According to various embodiments, the operation of predicting characteristics of a polymer composite material may be performed based on at least one deep learning algorithm. To describe in more detail, the operation of predicting characteristics of a polymer composite material is based on at least one deep learning model, and when inputting recipes, the properties of the polymer composite material predicted to be synthesized according to the input materials may be predicted based on the mixing ratio and properties of the materials.

According to an embodiment, the deep learning model may include a property prediction model trained to predict properties of a polymer composite material prepared for various properties of materials containing at least one polymer and a mixing ratio of the materials.

Here, the polymer composite material may be a material prepared using at least one polymer as at least a portion of various polymer composites, such as polymer blends, polymer copolymer, polymer nanocomposites, polymer interpenetrating network (IPN), or polymer metal composites.

In addition, the polymer which is the material of the polymer composite material may include at least one polymer among polypropylene (PP), polyethylene (PE), and polyethylene terephthalate (PET).

Here, the polymer may include at least one of a variety of recycled polymers, such as recycled polypropylene (rPP), recycled polyethylene (rPE), and recycled polyethylene terephthalate (rPET), as well as normal or virgin polymer as described above.

Hereinafter, various embodiments of the present disclosure will be described with reference to the accompanying drawings. However, the drawings attached to the present specification serve to further understand the technical idea together with the detailed description, such that the present disclosure should not be construed as being limited only to the illustrations of the drawings.

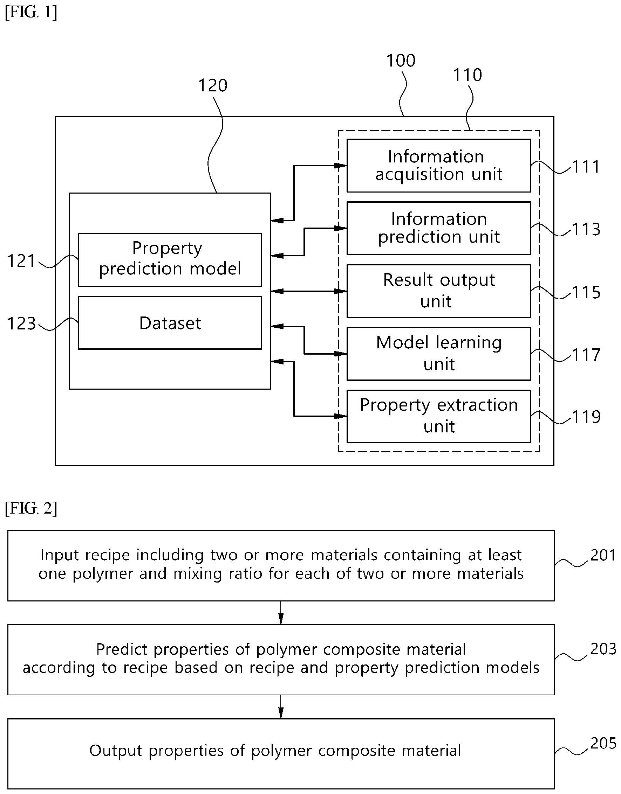

FIG. 1 is a block diagram illustrating the configuration of a device according to an embodiment. To describe in more detail, the device may be illustrated as a block diagram by dividing the detailed configuration according to functions thereof as shown in FIG. 1.

First, referring to FIG. 1, a device 100 may include: an information acquisition unit 111 configured to acquire recipes for synthesizing a polymer composite material in order to predict properties of the polymer composite material according to the recipes using the input recipes, and acquire a recipe including a plurality of materials and properties of each of the materials, and a mixing ratio of the materials from the acquired recipe; an information prediction unit 113 configured to predict the properties of the polymer composite material based on the acquired properties and mixing ratio of the materials; and a result output unit 115 configured to output the predicted properties of the polymer composite material.

Here, the information prediction unit 113 may predict the properties of the polymer composite material according to the properties of the materials and the mixing ratio of the materials acquired from the recipe through the pre-trained property prediction model 121.

In addition, the device 100 may further include a model learning unit 117 configured to perform training of the property prediction model 121.

According to various embodiments, the device 100 may include a storage unit 120 configured to store the property prediction model 121. In addition, at least one dataset 123 used to perform training of the property prediction model 121 may be stored in the storage unit 120.

Hereinafter, as an operation of the device 100 based on FIG. 1, operations of predicting the characteristics of a polymer composite material by the device 100 will be described with reference to FIGS. 2 to 8.

According to an embodiment, an operation of predicting the properties of the polymer composite material from the properties of materials included in the recipe using the property prediction model and an operation of training the property prediction model will be described with reference to FIGS. 2 to 4.

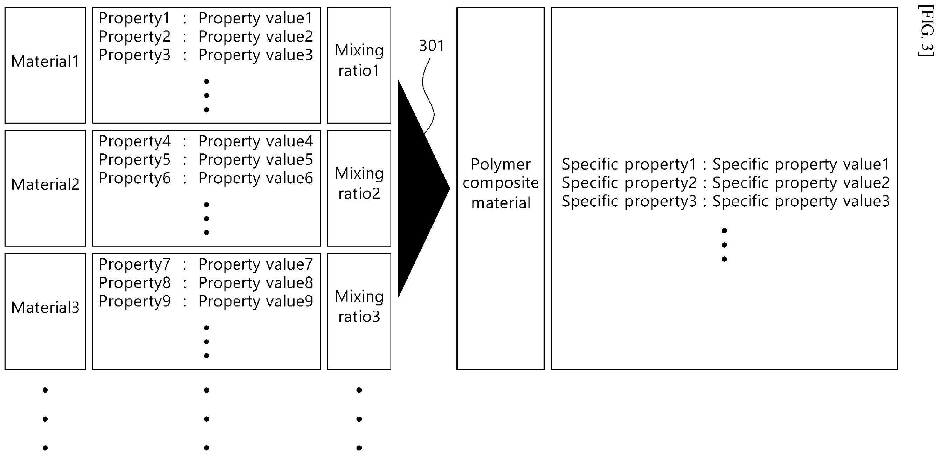

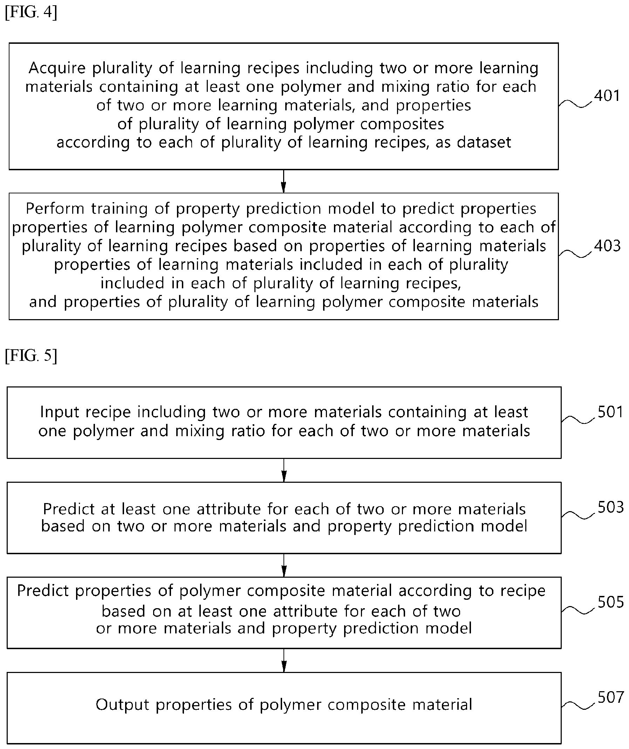

To this end, FIG. 2 is a flowchart illustrating the flow of an operation of predicting characteristics of a polymer composite material in the device according to an embodiment. FIG. 3 is a view schematically illustrating a property prediction model to output properties of the polymer composite material using properties of the materials included in the recipe as an input in the device according to an embodiment. In addition, FIG. 4 is a flowchart illustrating the flow of an operation of training the property prediction model based on a deep learning algorithm in the device according to an embodiment.

First, referring to FIG. 2, in step 201, the information acquisition unit 111 may input (or acquire) a recipe including two or more materials containing at least one polymer and a mixing ratio for each of the two or more materials.

According to an embodiment, the information acquisition unit 111 may acquire a specific recipe through at least one input unit (not shown) and/or communication unit (not shown) included in the device 100.

In one embodiment, the recipe may include materials capable of producing a specific polymer composite material or identification information corresponding to each of the materials among various materials for producing a polymer composite material, such as at least one polymer, a reinforcing agent (e.g., talc), and an additive (e.g., polyolefin ether).

At this time, if the recipe includes the identification information corresponding to each of the materials, the information acquisition unit 111 may acquire information on the materials corresponding to each of the identification information from the storage unit 120.

To this end, the storage unit 120 may store information on materials of the polymer composite material, identification information corresponding to the materials, and properties of each of the materials as a data table.

According to an embodiment, the properties of each of the materials stored in the storage unit 120 or the recipe may include at least one property of tensile strength, tensile failure, tearing strength, yield strength, flexural modulus, impact strength, elongation at break, heat distortion temperature, air permeability and shrinkage, and property values corresponding thereto.

To describe in more detail, the impact strength may include properties of dart impact strength and/or Izod impact strength. In addition, at least some of the tearing strength, yield strength, tensile failure, and elongation at break may include properties in a machine direction and/or properties in a transverse direction.

In describing embodiments of the present disclosure, when expressing the 'properties (or at least one property) of a specific material', it may be expressed including properties (or property items) and property values of the specific material such as property items (e.g., tensile strength) and property values (tensile strength value possessed by a specific polypropylene) of the specific material (e.g., specific polypropylene).

In addition, according to an embodiment, the mixing ratio is a relative weight (or mass) ratio for each of the materials (e.g., a weight ratio sum (WRS)), and a content for each material may be represented as a percent (%).

In addition, the recipe may include information on the properties of each of the included materials. However, the properties for each of the materials may be stored in the storage unit 120, and in this state, the information acquisition unit 111 may acquire the properties for each of the materials from the storage unit 120.

To describe the materials acquired from a specific recipe, properties of the materials, and a mixing ratio thereof in more detail with reference to FIG. 3, the information acquisition unit 111 may acquire various materials for forming a polymer composite material, including material 1, material 2, and material 3, from the acquired specific recipe.

At this time, the information acquisition unit 111 may acquire identification codes corresponding to materials from the recipe, and also acquire information on materials corresponding to each identification code from the storage unit 120.

Referring to FIG. 3, it illustrates that the materials included in the recipe are three materials, such as material 1, material 2, and material 3, but it is not limited thereto, and the recipe may also include at least one more or less material than three.

In addition, the information acquisition unit 111 may acquire the properties for each of the materials included in the recipe.

For example, the information acquisition unit 111 may acquire properties including property value 1 for property 1, property value 2 for property 2, and property value 3 for property 3 in material 1, properties including property value 4 for property 4, property value 5 for property 5, and property value 6 for property 6 in material 2, and properties including property value 7 for property 7, property value 8 for property 8, and property value 9 for property 9 in material 3.

Here, referring to FIG. 3, it illustrates that, for the properties included in each of the materials included in the recipe, three properties are included therein, but it is not limited thereto, and the properties for each of the materials may also include at least one more or less property than three.

Here, the expressions of properties 1 to 9 are expressions for describing the properties possessed by the materials, and two or more properties among properties 1 to 9 may represent the same property. Similarly, two or more among mixing ratios 1 to 3 may represent the same mixing ratio.

In addition, the information acquisition unit 111 may acquire the mixing ratio for each of the materials included in the recipe.

For example, the information acquisition unit 111 may acquire a mixing ratio 1 for material 1, a mixing ratio 2 for material 2, and a mixing ratio 3 for material 3. To describe in more detail, the mixing ratios of the materials included in the recipe may be included as a ratio possessed by each of the materials based on 100% of total materials, such as a mixing ratio of 20% for material 1, a mixing ratio of 30% for material 2, and a mixing ratio of 50% for material 3.

In step 203, the information prediction unit 113 may predict the properties of the polymer composite material according to the recipe based on the recipe and the property prediction models.

According to an embodiment, the information prediction unit 113 may process a plurality of properties for each of two or more materials acquired based on the recipe as an input to the property prediction model 121, and acquire properties of the polymer composite material according to the recipe as an output from the property prediction model 121.

To describe in more detail with reference to FIG. 3, the property prediction model according to the embodiments of FIGS. 2 to 4 may include a property-property prediction model (hereinafter, a property prediction model 301) trained to predict the properties of the polymer composite material according to the recipe based on the properties of the materials included in the recipe.

The information prediction unit 113 may process the materials included in the recipe (e.g., material 1, material 2, and material 3), the properties of the materials (e.g., properties including property value 1 for property 1, property value 2 for property 2, and property value 3 for property 3 in material 1, properties including property value 4 for property 4, property value 5 for property 5, and property value 6 for property 6 in material 2, and properties including property value 7 for property 7, property value 8 for property 8, and property value 9 for property 9 in material 3), and the mixing ratios for the materials (e.g., mixing ratio 1 for material 1, mixing ratio 2 for material 2, and mixing ratio 3 for material 3) as inputs to the property prediction model 301, and acquire specific properties of the polymer composite material according to the recipe (e.g., specific property value 1 for specific property 1, specific property value 2 for specific property 2, and specific property value 3 for a specific property 3).

Here, the specific property may include at least one property of melt index, tensile strength, tensile failure, tearing strength, yield strength, flexural modulus, impact strength, elongation at break, heat distortion temperature, air permeability and shrinkage.

Here, referring to FIG. 3, it is described that, for the properties of the polymer composite material output by the property prediction model 301, three specific properties are output, but it is not limited thereto, and the properties of the polymer composite material output by the property prediction model 301 may further include at least one more or less specific property than three.

When performing the embodiment according to FIGS. 2 and 3, the pre-trained property prediction model 301 of step 203 may be a deep learning model trained to output specific properties of the polymer composite material according to the recipe based on the properties of the materials included in the recipe.

In this regard, the training of the property prediction model 301 will be described in detail through FIG. 4.

First, the property prediction model 301 may be configured to perform training based on at least some of various deep learning algorithms which process structured data, such as TabNet, XGBoost, LightGBM, CatBoost, and deep neural network (DNN).

The property prediction model 301 may include a deep learning model configured to input recipes including a plurality of materials, properties for each of the materials, and a mixing ratio for the plurality of materials, and output properties of the polymer composite material corresponding to the recipes.

In step 401, the model learning unit 117 may acquire a plurality of learning recipes including two or more learning materials containing at least one polymer and a mixing ratio for each of the two or more learning materials, and properties of a plurality of learning polymer composite materials according to each of the plurality of learning recipes, as a dataset.

To describe in more detail, the dataset may include the plurality of learning recipes and the properties of the learning polymer composite materials according to each of the plurality of learning recipes. Here, each of the plurality of recipes may include information on two or more materials, properties of each of the two or more materials, and a mixing ratio of each of the two or more materials.

For example, to describe the dataset including information on a specific learning recipe and properties of the specific learning polymer composite material according to the specific learning recipe, it may include learning materials included in the specific learning recipe (e.g., material 4, material 5, and material 6), properties for the learning materials (e.g., properties including property value 10 for property 10, property value 11 for property 11, and property value 12 for property 12 in material 4, properties including property value 13 for property 13, property value 14 for property 14, and property value 15 for property 15 in material 5, and properties including property value 16 for property 16, property value 17 for property 17, and property value 18 for property 18 in material 6), and mixing ratios for the learning materials (e.g., mixing ratio 4 for material 4, mixing ratio 5 for material 5, and mixing ratio 6 for material 6).

The specific learning polymer composite material according to the specific learning recipe is a polymer composite material which is confirmed to be capable of being synthesized using the specific learning recipe, and may include properties possessed by the specific learning polymer composite material (e.g., properties including property value 19 for property 19, property value 20 for property 20, and property value 21 for property 21).

Here, it is described that the number of materials included in the learning recipe, the number of properties included in each of the materials, and the number of properties of the learning polymer composite material are three, respectively, but they are not limited thereto, and may be changed depending on the setting.

In addition, the expressions of properties 10 to 18 are expressions for describing the properties possessed by the materials, and two or more properties among properties 10 to 18 may represent the same property, and at least one property among properties 10 to 18 may represent the same property as at least one property among properties 1 to 9 and property 19 to property 21.

Similarly, two or more of the mixing ratios 4 to 6 may have the same mixing ratio, and at least one of the mixing ratios 4 to 6 may have the same value as at least one of the mixing ratios 1 to 3.

Here, a dataset 123 and/or data included in the dataset (e.g., recipes and properties of the polymer composite material corresponding to the recipes) may be received by the information acquisition unit 111 through an input unit or the communication unit.

Here, the properties of the polymer composite material may include at least some of melt index, tensile strength, tensile failure, tearing strength, yield strength, flexural modulus, impact strength, elongation at break, heat distortion temperature, air permeability and shrinkage.

In step 403, the model learning unit 117 may perform training of the property prediction model to predict the properties of the learning polymer composite material according to each of the plurality of learning recipes based on the properties of the learning materials included in each of the plurality of learning recipes, and the properties of the plurality of learning polymer composite materials.

To describe in more detail, the model learning unit 117 may perform training of the property prediction model 301 so as to, when inputting any one recipe, output the properties of the polymer composite material according to the corresponding recipe, based on the learning materials included in each of the plurality of learning recipes, properties of the learning materials, and properties of the plurality of learning polymer composite materials.

When performing training of the property prediction model 301, the model learning unit 117 may train the property prediction model 301 based on the specific properties of at least some specific materials included in the learning recipe, and specific properties of the learning polymer composite material.

To describe in more detail, the model learning unit 117 may extract, from each of the plurality of learning recipes, a first preset learning specific property among properties possessed by each of at least one learning specific material, and a second preset learning specific property among the properties of the learning polymer composite material.

To this end, the device 100 may further include a property extraction unit 119 for extracting the properties of the materials included in the recipes and/or specific properties from the properties of the polymer composite material.

Here, the specific material may include a polymer, talc, or a polyolefin elastomer.

Here, the model learning unit 117 and/or the property extraction unit 119 may extract a first learning specific property or a second learning specific property including at least some of melt index, tensile strength, tensile failure, tearing strength, yield strength, flexural modulus, impact strength, elongation at break, heat distortion temperature, air permeability and shrinkage.

Here, the second learning specific property may include at least one property among the specific properties of the polymer composite material to be predicted through the pre-trained property prediction model 121.

In addition, the model learning unit 117 and/or the property extraction unit 119 may extract the first learning specific property in different manners based on types of the learning specific material.

For example, when the learning specific material is polypropylene (PP), the model learning unit 117 and/or the property extraction unit 119 may extract at least one property among the melt index, flexural modulus, tensile strength, impact strength, shrinkage, and heat distortion temperature as a first feature.

When the learning specific material is polyethylene (PE), the model learning unit 117 and/or the property extraction unit 119 may extract at least one property among the impact strength, tearing strength, yield strength, tensile failure, and elongation at break as a second feature.

In addition, when the learning specific material is polyethylene terephthalate (PET), the model learning unit 117 and/or the property extraction unit 119 may extract at least one property among the air permeability, tensile strength, tearing strength, and elongation at break as a third feature.

Thereafter, the model learning unit 117 may perform training of the property prediction model for each of the plurality of learning recipes based on the plurality of learning recipes including the plurality of first learning specific properties and the second learning specific properties.

To describe in more detail, the model learning unit 117 may perform training of the property prediction model 301 to output properties including the second learning specific properties of the specific polymer composite material corresponding to the specific learning recipe by setting the first learning specific properties and/or the second learning specific properties as a region of interest, and using the specific learning recipe and the first learning specific properties of the specific learning recipe as inputs.

The information prediction unit 113 may perform an operation of predicting the properties of a polymer composite material for a recipe using the trained property prediction model 301 as described above.

Returning to FIG. 2 again, the operation of predicting the properties of the polymer composite material according to the input recipe using the trained property prediction model 301 will be described in more detail.

For example, in step 203, the information prediction unit 113 may process two or more materials included in the recipe, properties for each of the materials, and a mixing ratio of each of the materials as inputs to the property prediction model 301, and acquire properties of the polymer composite material from the property prediction model 301.

According to various embodiments, the information prediction unit 113 may extract at least one specific property among the plurality of properties possessed by each of at least one specific material among two or more materials included in the input recipe, and process it as an input to the property prediction model 301.

At this time, the information prediction unit 113 may predict the properties of the polymer composite material using the two or more materials and the at least one specific property of the at least one specific material as inputs to the property prediction model 301.

In step 205, the result output unit 115 may output the properties of the polymer composite material. To describe in more detail, the result output unit 115 may generate result information by including the recipe acquired in step 201 and the specific properties of the polymer composite material acquired from the property prediction model 301 corresponding to the acquired recipe, and output the result information.

According to various embodiments, the result output unit 115 may include at least some chemical formulas and/or at least some chemical structures of the polymer composite material predicted by the pre-trained property prediction model 121 corresponding to the recipe in the result information.

In addition, the result output unit 115 may display a portion corresponding to the specific properties of the predicted polymer composite material through at least some chemical formulas and/or at least some chemical structures. In this case, the result output unit 115 may display the portion corresponding to the specific property of the polymer composite material through highlighting or color change.

According to various embodiments, the result output unit 115 may transmit the result information to other preset devices through the communication unit 130.

When the operation of step 205 is completed, the result output unit 115 may terminate the procedures of embodiment in FIG. 2.

According to various embodiments, it is not limited to predicting the properties of the polymer composite material from the properties of the materials included in the recipe as described above, and the properties of the polymer composite material may also be predicted based on attributes of the materials.

In this regard, an operation of predicting the properties of the polymer composite material from the properties of the materials and the attributes of the materials included in the recipe using the property prediction model and an operation of training the property prediction model will be described with reference to FIGS. 5 to 8.

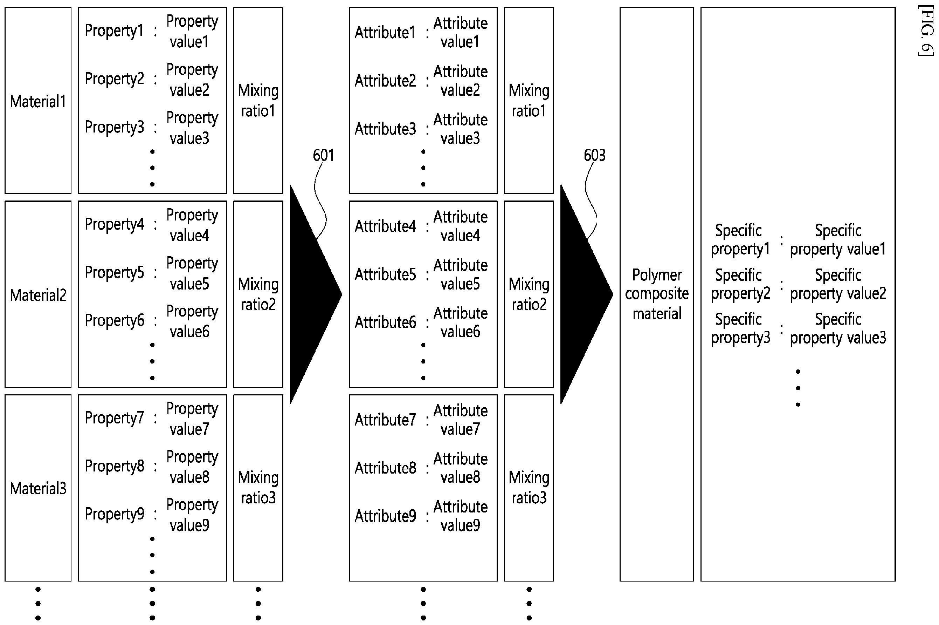

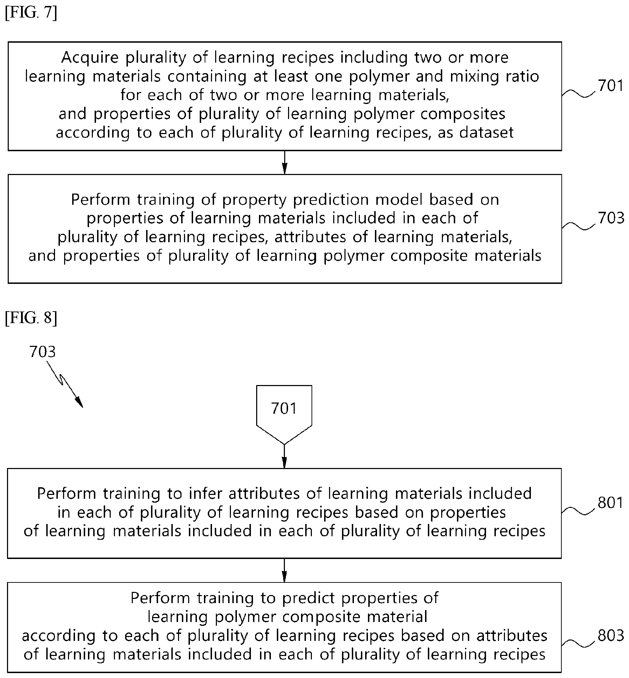

To this end, FIG. 5 is a flowchart illustrating the flow of an operation of predicting characteristics of the polymer composite material in the device according to an embodiment. FIG. 6 is a view schematically illustrating an operation of the property prediction model to predict attributes of the materials using the properties of the materials included in the recipe as an input, and output properties of the polymer composite material using the attributes of the materials as an input in the device according to an embodiment. FIG. 7 is a flowchart illustrating the flow of an operation of training the property prediction model based on the deep learning algorithm in the device according to an embodiment of the present disclosure. In addition, FIG. 8 is a flowchart illustrating the flow of the detailed operation of training the property prediction model based on the deep learning algorithm in the device according to an embodiment of the present disclosure.

First, referring to FIG. 5, in step 501, the information acquisition unit 111 may input (or acquire) a recipe including two or more materials containing at least one polymer and a mixing ratio for each of the two or more materials.

Step 501 may be performed identically or similarly to at least some procedures of step 201 in FIG. 2, unless specifically mentioned, and therefore will not be described in detail.

The materials acquired from the specific recipe, the properties of the materials, and the mixing ratio thereof have been described in detail with reference to FIG. 3, and therefore the operation of acquiring various materials, the properties of the materials, and the mixing ratio of the materials for forming the polymer composite material including material 1, material 2, and material 3 from the specific recipe by the information acquisition unit 111 will not be described.

Thereafter, the information prediction unit 113 may predict the attributes of the materials based on the properties of the materials acquired from the recipe, and may predict the properties of the polymer composite material based on the attributes of the materials.

For this purpose, to describe in more detail with reference to FIG. 6, the property prediction model 121 may include a property-attribute prediction model (hereinafter, a first prediction model 601) configured to predict attributes of the materials using the properties of materials as an input, and an attribute-property prediction model (hereinafter, a second prediction model 603) configured to predict specific properties of the polymer composite material using the attributes of the materials.

In step 503, the information prediction unit 113 may predict at least one attribute for each of the two or more materials based on the two or more materials and the first prediction model 601.

According to an embodiment, the information prediction unit 113 may process a plurality of properties for each of two or more materials acquired based on the recipe as an input to the first prediction model 601, and acquire a plurality of attributes for each of the two or more materials from the first prediction model 601 as an output.

The information prediction unit 113 may process materials included in the recipe (e.g., material 1, material 2, and material 3), properties of the materials (e.g., properties including property value 1 for property 1, property value 2 for property 2, and property value 3 for property 3 in material 1, properties including property value 4 for property 4, property value 5 for property 5, and property value 6 for property 6 in material 2, and properties including property value 7 for property 7, property value 8 for property 8, and property value 9 for property 9 in material 3), and mixing ratios for the materials (e.g., mixing ratio 1 for material 1, mixing ratio 2 for material 2, and mixing ratio 3 for material 3) as inputs to the first prediction model 601, and acquire attributes of the materials (e.g., attributes including attribute value 1 for attribute 1, attribute value 2 for attribute 2, and attribute value 3 for attribute 3 in material 1, attributes including attribute value 4 for attribute 4, attribute value 5 for attribute 5, and attribute value 6 for attribute 6 in material 2, and attributes including attribute value 7 for attribute 7, attribute value 8 for attribute 8, and attribute value 9 for attribute 9 in material 3).

Here, the attributes of the materials may include at least one attribute of syndiotactic PP content or molecular weight, atactic PP content or molecular weight, rubber content or molecular weight, type or content of reinforcing agent, PE content or molecular weight, type or content of copolymer, long-chain branch content, acid content, density, and degree of crystallization.

Here, referring to FIG. 6, it is described that, for each of the attributes of the materials output by the first prediction model 601, three attributes are output, but it is not limited thereto, and each of the attributes of the materials output by the first prediction model 601 may include at least one more or less attribute than three.

In step 505, the information prediction unit 113 may predict the properties of the polymer composite material according to the recipe based on the at least one attribute for each of the two or more materials and the property prediction model.

According to an embodiment, the information prediction unit 113 may process the attributes of the materials and the mixing ratio of the materials acquired from the first prediction model 601 as inputs to the second prediction model 603, and acquire the properties of the polymer composite material according to the recipe from the second prediction model 603 as an output.

The information prediction unit 113 may process the attributes for materials (e.g., attributes including attribute value 1 for attribute 1, attribute value 2 for attribute 2, and attribute value 3 for attribute 3 in material 1, attributes including attribute value 4 for attribute 4, attribute value 5 for attribute 5, and attribute value 6 for attribute 6 in material 2, and attributes including attribute value 7 for attribute 7, attribute value 8 for attribute 8, and attribute value 9 for attribute 9 in material 3) as an input to the second prediction model 603, and acquire specific properties of the polymer composite material according to the recipe (e.g., properties including specific property value 1 for specific property 1, specific property value 2 for specific property 2, and specific property value 3 for specific property 3).

Here, the specific property may include at least one property of melt index, tensile strength, tensile failure, tearing strength, yield strength, flexural modulus, impact strength, elongation at break, heat distortion temperature, air permeability and shrinkage.

Here, referring to FIG. 6, it is described that, for the properties of the polymer composite material output by the second prediction model 603, three specific properties are output, but it is not limited thereto, and the properties of the polymer composite material output by the second prediction model 603 may further include at least one more or less specific property than three.

When performing the embodiment according to FIGS. 5 and 6, the pre-trained first prediction model 601 of step 503 may be a deep learning model trained to output attributes of the materials based on the properties of the materials included in the recipe, and the pre-trained second prediction model 603 of step 505 may be a deep learning model trained to output specific properties of the polymer composite material according to the recipe based on the attributes of the materials.

In this regard, the training of the first prediction model 601 and the second prediction model 603 will be described in detail through FIGS. 7 and 8.

First, the first prediction model 601 and the second prediction model 603 may be configured to perform training based on at least some of various deep learning algorithms which process structured data, such as TabNet, XGBoost, LightGBM, CatBoost, and deep neural network (DNN).

In step 701, the model learning unit 117 may acquire a plurality of learning recipes including two or more learning materials containing at least one polymer and a mixing ratio for each of the two or more learning materials, and properties of a plurality of learning polymer composite materials according to each of the plurality of learning recipes, as a dataset.

To describe in more detail, the dataset may include the plurality of learning recipes and the properties of the learning polymer composite materials according to each of the plurality of learning recipes. Here, each of the plurality of recipes may include information on two or more materials, properties of each of the two or more materials, attributes of each of the two or more materials, and a mixing ratio of each of the two or more materials.

For example, to describe the dataset including information on a specific learning recipe and properties of the specific learning polymer composite material according to the specific learning recipe, it may include learning materials included in the specific learning recipe (e.g., material 4, material 5, and material 6), properties for the learning materials (e.g., properties including property value 10 for property 10, property value 11 for property 11, and property value 12 for property 12 in material 4, properties including property value 13 for property 13, property value 14 for property 14, and property value 15 for property 15 in material 5, and properties including property value 16 for property 16, property value 17 for property 17, and property value 18 for property 18 in material 6), attributes for the learning materials (e.g., attributes including attribute value 10 for attribute 10, attribute value 11 for attribute 11, and attribute value 12 for attribute 12 in material 4, attributes including attribute value 13 for attribute 13, attribute value 14 for attribute 14, and attribute value 15 for attribute 15 in material 5, and attributes including attribute value 16 for attribute 16, attribute value 17 for attribute 17, and attribute value 18 for attribute 18 in material 6), and mixing ratios for the learning materials (e.g., mixing ratio 4 for material 4, mixing ratio 5 for material 5, and mixing ratio 6 for material 6).

The specific learning polymer composite material according to the specific learning recipe is a polymer composite material which is confirmed to be capable of being synthesized using the specific learning recipe, and may include properties possessed by the specific learning polymer composite material (e.g., properties including property value 19 for property 19, property value 20 for property 20, and property value 21 for property 21).

Here, it is described that the number of materials included in the learning recipe, the number of properties included in each of the materials, and the number of properties of the learning polymer composite material are three, respectively, but they are not limited thereto, and may be changed depending on the setting.

In addition, the expressions of properties 10 to 18 are expressions for describing the properties possessed by the materials, and two or more properties among properties 10 to 18 may represent the same property, and at least one property among properties 10 to 18 may represent the same property as at least one property among properties 1 to 9 and property 19 to property 21.

Similarly, two or more of the mixing ratios 4 to 6 may have the same mixing ratio, and at least one of the mixing ratios 4 to 6 may have the same value as at least one of the mixing ratios 1 to 3.

Here, the dataset 123 and/or data included in the dataset (e.g., recipes and properties of the polymer composite material corresponding to the recipes) may be received by the information acquisition unit 111 through an input unit or the communication unit.

In step 703, the model learning unit 117 may perform training of the property prediction model based on properties of the learning materials included in each of the plurality of learning recipes, attributes of the learning materials, and the properties of the plurality of learning polymer composite materials.

In this regard, the operation of training the first prediction model 601 and the second prediction model 603 will be described in more detail with reference to FIG. 8. At least some procedures of steps 801 and/or 803 in FIG. 8 may be executed in step 703 of FIG. 7.

In step 801, the model learning unit 117 may perform training of the first prediction model 601 to infer attributes of the learning materials included in each of the plurality of learning recipes based on the properties of the learning materials included in each of the plurality of learning recipes.

To describe in more detail, when inputting any one recipe based on the learning materials included in each of the plurality of learning recipes and the properties of the learning materials, the model learning unit 117 may perform training of the first prediction model 601 to output the attributes of the materials included in the recipe.

When performing training of the first prediction model 601, the model learning unit 117 may train the first prediction model 601 based on the specific properties of at least some specific materials included in the learning recipes.

To describe in more detail, the model learning unit 117 may extract, from each of the plurality of learning recipes, a first preset learning specific property among properties possessed by each of at least one learning specific material, and a second preset learning specific property among the properties of the learning polymer composite material.

To this end, the device 100 may further include a property extraction unit 119 for extracting the properties of the materials included in the recipes and/or specific properties from the properties of the polymer composite material.

Here, the specific material may include a polymer, talc, or a polyolefin elastomer.

Here, the model learning unit 117 and/or the property extraction unit 119 may extract a first learning specific property or a second learning specific property including at least some of melt index, tensile strength, tensile failure, tearing strength, yield strength, flexural modulus, impact strength, elongation at break, heat distortion temperature, air permeability and shrinkage.

Here, the second learning specific property may include at least one property among the specific properties of the polymer composite material to be predicted through the pre-trained property prediction model 121.

In addition, the model learning unit 117 and/or the property extraction unit 119 may extract the first learning specific property in different manners based on types of the learning specific material.

For example, when the learning specific material is polypropylene (PP), the model learning unit 117 and/or the property extraction unit 119 may extract at least one property among the melt index, flexural modulus, tensile strength, impact strength, shrinkage, and heat distortion temperature as a first feature.

When the learning specific material is polyethylene (PE), the model learning unit 117 and/or the property extraction unit 119 may extract at least one property among the impact strength, tearing strength, yield strength, tensile failure, and elongation at break as a second feature.

In addition, when the learning specific material is polyethylene terephthalate (PET), the model learning unit 117 and/or the property extraction unit 119 may extract at least one property among the air permeability, tensile strength, tearing strength, and elongation at break as a third feature.

Thereafter, the model learning unit 117 may perform training of the first prediction model 601 to infer attributes of the learning materials included in each of the plurality of learning recipes based on the first learning specific properties included in each of the plurality of learning recipes.

To describe in more detail, the model learning unit 117 may perform training of the first prediction model 601 to output attributes for at least some materials including specific materials among the materials included in the specific learning recipe by setting the first learning specific property as a region of interest, and using the specific learning recipe and the first learning specific property of the specific learning recipe as inputs.

In step 803, the model learning unit 117 may perform training of the second prediction model 603 to predict properties of the learning polymer composite material according to each of the plurality of learning recipes based on the attributes of the learning materials included in each of the plurality of learning recipes.

To describe in more detail, when inputting properties of at least some materials included in any one recipe based on the learning materials included in each of a plurality of learning recipes and attributes of the learning materials, the model learning unit 117 may perform training of the second prediction model 603 to output properties of the polymer composite material according to the recipes.

When performing training of the second prediction model 603, the model learning unit 117 may train the second prediction model 603 based on the plurality of materials included in the learning recipe and attributes of at least some specific materials.

Thereafter, the model learning unit 117 may perform training of the second prediction model 603 to predict the second specific property of the learning polymer composite material according to each of the plurality of learning recipes based on the attributes of the learning materials included in each of the plurality of learning recipes, and the second preset learning specific property among the properties of the learning polymer composite material.

To describe in more detail, the model learning unit 117 may perform training of the second prediction model 603 to output properties including the second learning specific property of the specific polymer composite material corresponding to the specific learning recipe by setting the second learning specific property as a region of interest, and using the specific learning recipe, the attributes for at least some including specific materials among the materials included in the specific learning recipe, and the second learning specific property of the specific learning recipe as inputs.

The information prediction unit 113 may perform an operation of predicting the properties of the polymer composite material for the recipe using the property prediction model 121 including the first prediction model 601 and the second prediction model 603, which are trained as described above.

Returning to FIG. 5 again, the operation of predicting the properties of the polymer composite material according to the input recipe using the trained first prediction model 601 and/or second prediction model 603 will be described in more detail.

For example, in step 503, the information prediction unit 113 may process two or more materials included in the recipe, properties for each of the materials, and a mixing ratio of each of the materials as inputs to the first prediction model 601, and acquire attributes for at least some of the materials included in the recipe from the first prediction model 601.

Here, the information prediction unit 113 may extract at least one specific property among the plurality of properties possessed by each of at least one specific material among two or more materials included in the input recipe, and process it as an input to the first prediction model 601.

At this time, the information prediction unit 113 may predict attributes for at least some of the materials included in the recipe using the two or more materials and the at least one specific property for the at least one specific material as inputs to the first prediction model 601.

Thereafter, the information prediction unit 113 may perform the operation of step 505 as described above based on the attributes of the materials acquired from the first prediction model 601 and the input recipe.

In step 507, the result output unit 115 may output the properties of the polymer composite material. To describe in more detail, the result output unit 115 may generate result information by including the recipe acquired in step 201 and the specific properties of the polymer composite material acquired from the property prediction model 301 corresponding to the acquired recipe, and output the result information. Here, the result output unit 115 may further include the predicted attributes of the materials included in the recipe in the result information.

According to various embodiments, the result output unit 115 may include at least some chemical formulas and/or at least some chemical structures of the polymer composite material predicted by the pre-trained property prediction model 121 corresponding to the recipe in the result information.

In addition, the result output unit 115 may display the portion corresponding to the specific property of the predicted polymer composite material through at least some chemical formulas and/or at least some chemical structures. In this case, the result output unit 115 may display the portion corresponding to the specific property of the polymer composite material through highlighting or color change.

According to various embodiments, the result output unit 115 may transmit the result information to other preset devices through the communication unit 130.

When the operation of step 507 is completed, the result output unit 115 may terminate the procedures of embodiment in FIG. 2.

According to various embodiments, the method for predicting characteristics of a polymer composite material and the device thereof may provide an environment capable of quickly checking changes in the properties of the polymer composite material prepared during synthesis only by changing the materials and the mixing ratio of the materials.

According to various embodiments, the method for predicting characteristics of a polymer composite material and the device thereof allow users to predict the characteristics of the polymer composite material prepared during synthesis according to the mixing ratio of the materials included in the recipes without performing the material synthesis and property measurement processes for the polymer composite material, thereby significantly reducing the time and costs required for development of materials.

According to the above description, two or more of the information acquisition unit 111, the information prediction unit 113, the result output unit 115, the model learning unit 117, and the property extraction unit 119 may be formed as one module, or each unit may be formed as a separate module. In this case, each module may include at least one processor.

According to various embodiments, the functions of various embodiments described as being performed by the device 100 are operations processed through the processing unit 110 of the device 100, and may be performed by organically being connected to the device 100 and/or components of the device connected to the device 100.

To this end, at least some of the information acquisition unit 111, the information prediction unit 113, the result output unit 115, the model learning unit 117, and the property extraction unit 119 may be formed to be included in the processing unit 110 as shown in FIG. 1.

The processing unit 110 includes at least one processor, and may process data received from a battery connected to the device 100 through at least one program (application, tool, plugin, software, etc., hereinafter referred to as a battery diagnostic program).

The storage unit 120 may store various data processed by at least one component of the device 100 (e.g., the processing unit 110, the communication unit or the like). The data may include, for example, a program for processing control commands, data processed through the program, or input data and output data related thereto.

The communication unit (not shown) may support establishment of a wired communication channel or establishment of a wireless communication channel inside the device 100 and/or between the device 100 and at least one other device (e.g., the user device or a server), and performing communication through the established communication channel.

The input/output unit may include or be connected to at least some of an input unit (not shown) for inputting data, such as a keyboard, mouse, or touch pad, and an output unit (not shown) for outputting data, such as a display unit (e.g., display), speaker, or driving unit.

According to various embodiments of the present disclosure, the device 100 or user device may include at least some of the functions in the range of all information and communication devices, including a mobile terminal, a multimedia terminal, a wired terminal, a fixed terminal, and an internet protocol (IP) terminal.

The device 100 is a device for processing the control commands, and may include at least some of the functions of a workstation or a large-scale database, or may be connected therewith through communication.

As described above, although the embodiments have been described with reference to the limited drawings, it will be apparent to those skilled in the art that various modifications and alternations may be applied thereto based on the various embodiments.

For example, adequate effects or results may be achieved even if the foregoing processes and methods are carried out in different order than those described above, and/or the above-described elements, such as systems, structures, devices, or circuits, are combined or coupled in different forms and modes than those described above, or substituted or switched with other components or equivalents.

In particular, when describing with reference to the flowchart, it is described that a plurality of steps are configured and the steps are sequentially executed in a designated order, but it is not necessarily limited to the designated order.

In other words, executing by changing or deleting at least some of the steps described in the flowchart or adding at least one step is applicable as an embodiment, and executing one or more steps in parallel may also be applicable as an embodiment. That is, it is not limited to that the steps are necessarily operated in a time-series order, and should be included in various embodiments of the present disclosure.

Therefore, other implements, other embodiments, and equivalents to claims are within the scope of claims to be described below.

## Claims

1. A method for predicting characteristics of a polymer composite material, the method comprising:
inputting a recipe including two or more materials containing at least one polymer and a mixing ratio for each of the two or more materials;
predicting properties of the polymer composite material according to the recipe based on a property prediction model (121); and
outputting the properties of the polymer composite material.

2. The method according to claim 1, wherein training of the property prediction model (121) is performed through the steps of
acquiring a plurality of learning recipes including two or more learning materials containing at least one polymer and a mixing ratio for each of the two or more learning materials, and properties of a plurality of learning polymer composite materials according to each of the plurality of learning recipes, as a dataset (123); and
performing training of the property prediction model (121) to predict properties of the learning polymer composite material according to each of the plurality of learning recipes based on the properties of the learning materials included in each of the plurality of learning recipes, and the properties of the plurality of learning polymer composite materials.

3. The method according to claim 1 or 2, wherein the step of performing training of the property prediction model (121) comprises:
extracting, from each of the plurality of learning recipes, a first preset learning specific property among properties possessed by each of at least one learning specific material, and a second preset learning specific property among the properties of the learning polymer composite material; and
performing training of the property prediction model (121) for each of the plurality of learning recipes based on the plurality of learning recipes including the plurality of first learning specific properties and the second learning specific properties,
wherein the specific material includes at least one material of a polymer, talc, and a polyolefin elastomer.

4. The method according to anyone of the preceding claims, wherein the step of predicting properties of the polymer composite material comprises:
extracting at least one specific property among a plurality of properties possessed by each of at least one specific material among the two or more materials; and
predicting properties of the polymer composite material using the two or more materials and the at least one specific property of the at least one specific material as inputs to the property prediction model (121).

5. A method for predicting characteristics of a polymer composite material, the method comprising:
inputting a recipe including two or more materials containing at least one polymer and a mixing ratio for each of the two or more materials;
predicting at least one attribute for each of the two or more materials based on the two or more materials and a property prediction model (121);
predicting properties of the polymer composite material according to the recipe based on the at least one attribute for each of the two or more materials and the property prediction model (121); and
outputting the properties of the polymer composite material.

6. The method according to claim 5, wherein training of the property prediction model (121) is performed through the steps of
acquiring a plurality of learning recipes including two or more learning materials containing at least one polymer and a mixing ratio for each of the two or more learning materials, and properties of a plurality of learning polymer composite materials according to each of the plurality of learning recipes, as a dataset (123); and
performing training of the property prediction model (121) based on properties of the learning materials included in each of the plurality of learning recipes, attributes of the learning materials, and the properties of the plurality of learning polymer composite materials.

7. The method according to claim 6, wherein the step of performing training of the property prediction model (121) comprises:
training to infer attributes of the learning materials included in each of the plurality of learning recipes based on the properties of the learning materials included in each of the plurality of learning recipes; and
training to predict the properties of the learning polymer composite material according to each of the plurality of learning recipes based on the attributes of the learning materials included in each of the plurality of learning recipes.

8. The method according to claim 7, wherein the step of performing training of the property prediction model (121) further comprises
extracting, from each of the plurality of learning recipes, a first preset learning specific property among properties possessed by each of at least one learning specific material, and a second preset learning specific property among the properties of the learning polymer composite material,
wherein the step of training to infer attributes of the learning materials performs training to infer attributes of the learning materials included in each of the plurality of learning recipes based on the first learning specific property included in each of the plurality of learning recipes,
the step of training to predict properties of the learning polymer composite material performs training to predict the second specific property of the learning polymer composite material according to each of the plurality of learning recipes based on the attributes of the learning materials included in each of the plurality of learning recipes, and
the specific material includes at least one material of a polymer, talc, and a polyolefin elastomer.

9. The method according to claim 5, wherein the step of predicting properties of the polymer composite material comprises:
extracting at least one specific property among a plurality of properties possessed by each of at least one specific material among the two or more materials; and
predicting properties of the polymer composite material using the two or more materials and the at least one specific property of the at least one specific material as inputs to the property prediction model (121).

10. A device for predicting characteristics of a polymer composite material, the device comprising:
an information input unit (111) configured to input a recipe including two or more materials containing at least one polymer and a mixing ratio for each of the two or more materials;
an information prediction unit (113) configured to predict properties of the polymer composite material according to the recipe based on a recipe and property prediction model (121); and
a result output unit (115) configured to output the properties of the polymer composite material.

11. The device according to claim 10, further comprising a model learning unit (117) configured to:
acquire a plurality of learning recipes including two or more learning materials containing at least one polymer and a mixing ratio for each of the two or more learning materials, and properties of a plurality of learning polymer composite materials according to each of the plurality of learning recipes, as a dataset (123); and
perform training of the property prediction model (121) to predict the properties of the learning polymer composite material according to each of the plurality of learning recipes based on the properties of the learning materials included in each of the plurality of learning recipes, and the properties of the plurality of learning polymer composite materials,
wherein the property prediction model (121) is trained through the model learning unit.

12. The device according to claim 11, wherein the model learning unit is configured to:
extract, from each of the plurality of learning recipes, a first preset learning specific property among properties possessed by each of at least one learning specific material, and a second preset learning specific property among the properties of the learning polymer composite material; and
perform training of the property prediction model (121) for each of the plurality of learning recipes based on the plurality of learning recipes including the plurality of first learning specific properties and the second learning specific properties,
wherein the specific material includes at least one material of a polymer, talc, and a polyolefin elastomer.

13. The device according to anyone of claims 10-12, wherein the information prediction unit (113) is configured to:
extract at least one specific property among a plurality of properties possessed by each of at least one specific material among the two or more materials; and
predict the properties of the polymer composite material using the two or more materials and the at least one specific property of the at least one specific material as inputs to the property prediction model (121).

14. A device for predicting characteristics of a polymer composite material, the device comprising:
an information input unit (111) configured to input a recipe including two or more materials containing at least one polymer and a mixing ratio for each of the two or more materials;
an information prediction unit (113) configured to predict at least one attribute for each of the two or more materials based on the two or more materials and a property prediction model (121), and predict properties of the polymer composite material according to the recipe based on the at least one attribute for each of the two or more materials and the property prediction model (121); and
a result output unit (115) configured to output the properties of the polymer composite material.

15. The device according to claim 14, wherein the property prediction model (121) comprises:
a property-attribute prediction model configured to predict the at least one attribute; and
an attribute-property prediction model (121) configured to predict the properties of the polymer composite material.
